Europäisches Patentamt
European Patent Office
Office européen des brevets

(19)

(11) Publication number: **0 236 948**
**A2**

(12) **EUROPEAN PATENT APPLICATION**

(21) Application number: 87103127.4

(22) Date of filing: 05.03.87

(51) Int. Cl.$^3$: C 07 K 5/02
C 12 P 21/02
//A61K37/64, (C12P21/02,
C12R1:465)

(30) Priority: 07.03.86 JP 49589/86

(43) Date of publication of application:
16.09.87 Bulletin 87/38

(84) Designated Contracting States:
CH DE FR GB IT LI NL

(71) Applicant: KITASATO KENKYUSHO
9-1, Shirokane 5 chome Minato-ku
Tokyo-to(JP)

(72) Inventor: Omura, Satoshi
5-12-7, Seta Setagaya-ku
Tokyo 158(JP)

(72) Inventor: Tanaka, Haruo
3-10-8, Morino
Machida-shi Tokyo 194(JP)

(72) Inventor: Imamura, Nobutaka
102 Green House Todoroki 8-26-2, Todoroki
Setagaya-ku Tokyo 158(JP)

(74) Representative: von Kreisler, Alek, Dipl.-Chem. et al,
Deichmannhaus am Hauptbahnhof
D-5000 Köln 1(DE)

(54) **Novel compounds WK-142 and process for preparing the same.**

(57) Novel compounds WK-142 having the following formula:

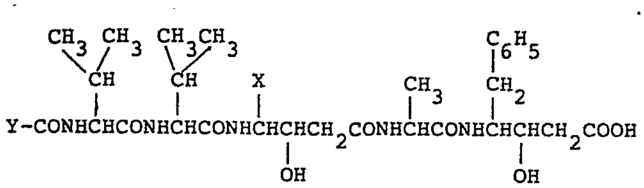

wherein X stands for a benzyl group or an isobutyl group and Y stands for an alkyl group, or pharmaceutically acceptable salts thereof, and a cultivating method for preparing the same are disclosed.

The novel compounds have acid protease-inhibiting activity.

EP 0 236 948 A2

# NOVEL COMPOUNDS WK-142 AND PROCESS FOR PREPARING THE SAME

## BACKGROUND OF THE INVENTION

### FIELD OF THE INVENTION

This invention relates to novel compounds WK-142 and pharmaceutically acceptable salts thereof and to a process for preparing such compounds. The compounds and their salts according to the present invention are useful as acid protease inhibitors.

### DESCRIPTION OF THE PRIOR ART

As inhibitors against acid proteases such as pepsin, cathepsin renin and chymosion, there are known pepstatin, diazoacetylnorleucine methyl ester, etc. Pepstatin, for example, is known to serve to suppress ulcers by inhibiting pepsin.

## SUMMARY OF THE INVENTION

The present invention is based on the finding that a certain class of microorganisms isolated by the present inventors from a soil has an ability to produce novel compounds and that the compounds have inhibiting activity against acid proteases.

The present invention is aimed at the provision of novel compounds and physiologically acceptable salts thereof and of a process for preparing such compounds.

In accordance with the present invention there is provided novel compounds of the following general formula:

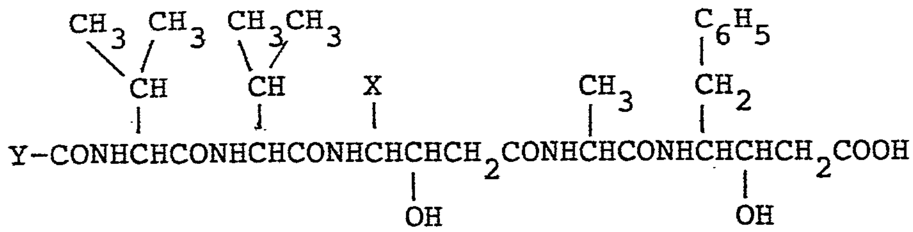

(I)

wherein X stands for a benzyl group or an isobutyl group and Y stands for an alkyl group, or pharmaceutically acceptable salts thereof.

Alkyl group expressed as Y, is an alkyl of 1 to 18 carbon atoms, more preferably 1 to 6 carbon atoms. Examples of alkyl group include n-propyl, isopropyl and isobutyl.

The novel compounds are named as WK-142 by the present inventors. The compounds of the present invention may be prepared by a method which includes culturing in a culture medium a microorganism which belongs to the genus Streptomyces and which is capable of producing the compounds WK-142, so that the compounds WK-142 are produced and accumulated in the culture, and collecting the compounds WK-142 from the culture.

BRIEF DESCRIPTION OF THE DRAWINGS

Fig. 1 is a UV absorption spectrum of the compound A, measured in methanol;

Fig. 2 is an IR absorpition spectrum measured by KBr disc method; and

Fig. 3 is a proton NMR spectrum measured in DMSO-$D_6$.

DETAILED DESCRIPTION OF THE INVENTION

Compounds WK-142-A, B, C and D which are specific

examples of the compounds of the present invention have the following physicochemical properties:

WK-142-A

(1) Colorless needle crystals

(2) Elementary analysis

$C_{37}H_{61}N_5O_9$

Calculated: C 61.73; H 8.54; N 9.73 %

Found : C 62.02; H 8.65; N 9.65 %

(3) Molecular weight and molecular formula:

A molecular ion peak was observed at m/z 733 on the mass spectrum of a methyl ester obtained by esterification with diazomethane. From the analysis of the dehydration peak m/z 715 by high-resolution mass spectrum (Found 715.451; Calculated 715.452), the molecular formula of the methyl ester was found to be $C_{38}H_{63}N_5O_9$. Thus, the molecular formula of the compound A is $C_{37}H_{61}N_5O_9$ and the molecular weight thereof is 719.93.

(4) UV absorption spectrum:

The UV absorption spectrum of the compound A is shown in Fig. 1. Characteristic absorption maxima are observed at 252, 258, 262 and 267 nm.

(5) IR absorption spectrum:

The IR spectrum of the compound A measured according to the KBr disc method is shown in Fig. 2. The wave numbers of main absorption maxima are as follows: 3300, 2960, 1650, 1630, 1620 and 1540 ($cm^{-1}$)

(6) NMR spectrum:

The $^{1}H$ NMR spectrum measured in DMSO-$D_6$ by Varian XL-400 NMR spectrometer (400 MHz) is shown in Fig. 3.

(7) Color reaction:

| | |
|---|---|
| Rydon -Smith | positive |
| Potassium permanganate | positive |
| Sulfuric acid | positive |
| Ninhydrin | negative |

(8) The structure of the compound A is as shown below:

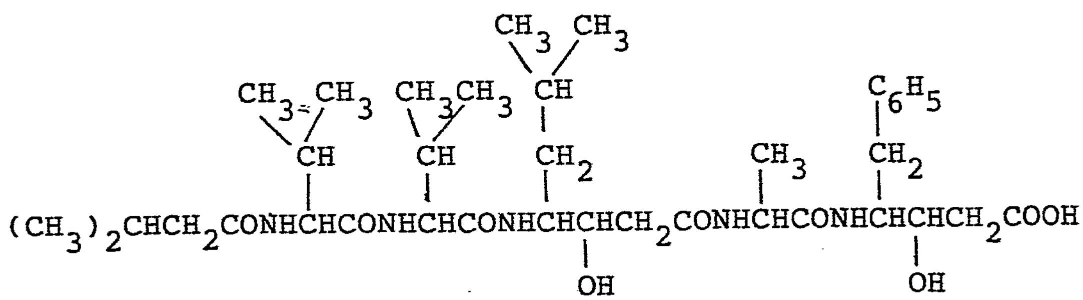

(Ia)

The structure Ia shown above is determined based on the results of comparison between the above-described physico-chemical properties and those of similar known compounds.

WK-142-B

(1) Colorless needle crystals

(2) Molecular weight and molecular formula:

A molecular ion peak was observed at *m/z* 767 on the mass spectrum of a methyl ester obtained by esterification with diazomethane. From the analysis of the dehydration ($-2H_2O$) peak at *m/z* 731 by high-resolution mass spectrum (Found 731.428; Calculated 731.425), the molecular formula of the methyl ester was found to be $C_{41}H_{61}N_5O_9$. Thus, the

compound B has a molecular formula of $C_{40}H_{59}N_5O_9$ and a molecular weight of 753.94.

(3) UV absorption spectrum:

A neutral solution gives characteristic absorption maxima at 252, 258, 262 and 267 nm.

(4) Color reaction:

| | |
|---|---|
| Rydon-Smith | positive |
| Potassium permanganate | positive |
| Sulfuric acid | positive |
| Ninhydrin | negative |

(5) The structure of the compound B is as shown below:

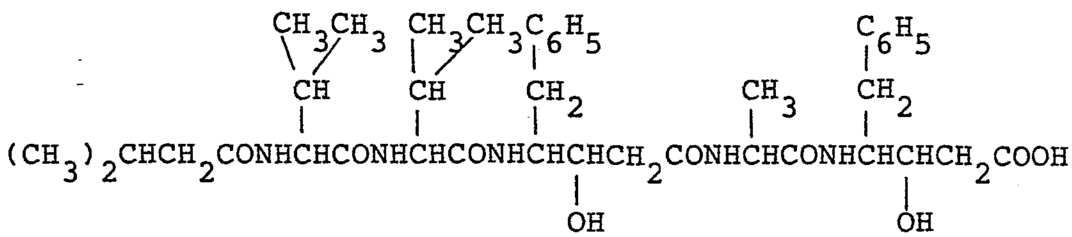

(Ib)

The structure Ib shown above is determined based on the results of comparison between the above-described physico-chemical properties and those of similar known compounds.

WK-142-C

(1) Colorless needle crystals

(2) Molecular weight and molecular formula:

A molecular ion peak was observed at m/z 719 on the mass spectrum of a methyl ester obtained by esterification with diazomethane. From the high-resolution mass spectrum of the ion peak (Found 719.454; Calculated 719.447), the

molecular formula of the methyl ester was found to be $C_{37}H_{61}N_5O_9$. Thus, the compound C has a molecular formula of $C_{36}H_{59}N_5O_9$ and a molecular weight of 705.90.

(3) UV absorption spectrum:

A neutral solution gives characteristic absorption maxima at 252, 258, 262 and 267 nm.

(4) Color reaction:

| | |
|---|---|
| Rydon-Smith | positive |
| Potassium permanganate | positive |
| Sulfuric acid | positive |
| Ninhydrin | negative |

(5) The structure of the compound C is as follows:

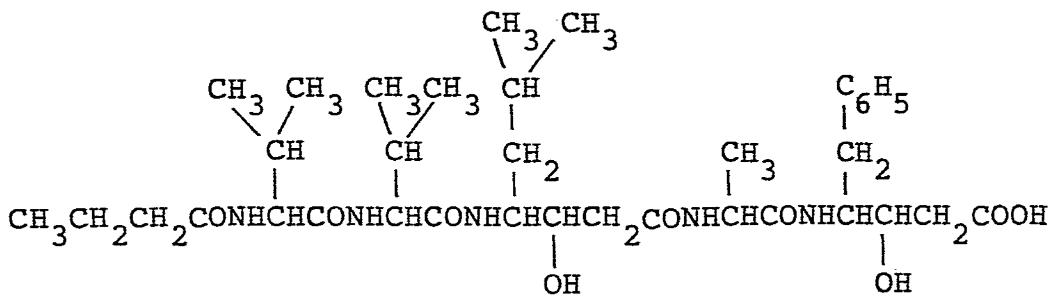

(Ic)

The structure Ic shown above is determined based on the results of comparison between the above-described physico-chemical properties and those of similar known compounds.

WK-142-D

(1) Colorless needle crystals

(2) Molecular weight and molecular formula:

A molecular ion peak was observed at m/z 719 on the mass spectrum of a methyl ester obtained by esterification

with diazomethane. From the high-resolution mass spectrum of the ion peak (Found 719.438; Calculated 719.447), the methyl ester was found to have a molecular formula of $C_{37}H_{61}N_5O_9$. Thus, the compound D has a molecular formula of $C_{36}H_{59}N_5O_9$ and a molecular weight of 705.90.

(3) UV absorption spectrum:

A neutral solution gives characteristic absorption maxima at 252, 258, 262 and 267 nm.

(4) Color reaction:

| | |
|---|---|
| Rydon -Smith | positive |
| Potassium permanganate | positive |
| Sulfuric acid | positive |
| Ninhydrin | negative |

(5) The structure of the compound D is as follows:

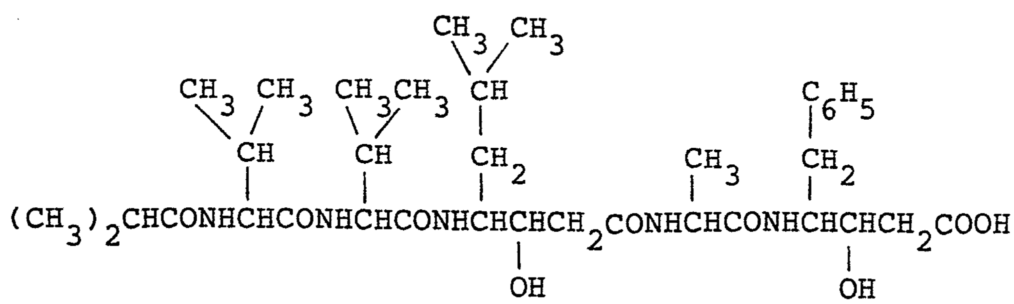

(Id)

The structure Id shown above is determined based on the results of comparison between the above-described physico-chemical properties and those of similar known compounds.

Acid Protease-Inhibiting Activity:

(1) Pepsin-Inhibiting Activity:

The pepsin-inhibiting activity was measured in terms of $IC_{50}$ value according to the method of Anson et al

[Journal of General Physiology 22, 79 (1938)] using pepsin from coats of pig's stomach (P7012 manufactured by Sigma Chemical Inc.). As a result, the $IC_{50}$ value of the compound A was found to be $1x10^{-8}M$. The pepsin-inhibiting activity of a known acid protease inhibitor, pepstatin A, was also measured in the similar manner to give an $IC_{50}$ value of $3x10^{-9}M$.

(2) Renin-Inhibiting Activity:

The renin-inhibiting activity was measured in terms of $IC_{50}$ in accordance with the method of Ikeda et al [Journal of Clinical Endoclinology and Metabolism 54, 423 (1982)] using renin obtained from pig's kidney. As a result, the $IC_{50}$ value of the compound A was found to be $7x10^{-8}M$. The renin-inhibiting activity of a known acid protease inhibitor, pepstatin A, was measured in the similar manner to give $IC_{50}$ value of $4.7x10^{-7}M$.

Among acid proteases, renin nis known to be the only enzyme that is hard to be inhibited. For example, the above results indicate that the ratio of the pepsin-inhibiting activity to renin-inhibiting activity of the known acid protetase, pepstatin A, is about 60:1. On the other hand, the activity ratio in to case of the compound A is 7:1. Thus, the compound A is regarded to be an acid protease inhibitor with a strong inhibiting activity against renin.

Toxicity:

Acid protease inhibitors are generally known to be

low in toxicity and the compounds WK-142 are also considered to have low toxicity.

Since the compounds WK-142 according to the present invention exhibit acid protease-inhibiting activity, ulcer is likely to be supressed by the compounds.

The compounds WK-142 may be prepared by a method as follows:

The strain of microorganism used for the production of the novel compounds WK-142 of this invention may be, for example, strain Streptomyces sp. WK-142 isolated from the soil obtained at Suzuki, Shimoda-shi, Shizuoka-ken by the present inventors.

The mycetological characteristics of this strain are as shown below:

(I) Morphological characteristics:

The vegetative mycelia develop abundantly on various agar media and do not show fragmentation. The aerial mycelia grow abundantly on starch-inorganic slats agar, yeast extract-malt extract agar, etc. and are white or gray in color. The microscopic observation reveals that the aerial mycelia are spiral and there are spore chains with more than 20 spores. Each spore has a columnar shape with a size of 0.9x0.5 μm and a smooth surface. No sclerotia, sporangia or zoospores are observed.

(II) Characteristics on various media:

The producing strain was assayed for its cultural characteristics according to the method of E. B. Schirling

and D. Gottlieb [International Journal of Systematic Biotechnology 16, 313 (1966)] to give the results summarized in Table below. The color tone was determined using the Color Harmony Mannual, 4th Ed. (published by Container Corporation of America, Chicago, 1958) as standard colors. In the Table below, color numbers are indicated in parentheses immediately after the color names. Except otherwise specifically noted, the results in Table below were those obtained after 2 weeks cultivation at 27°C in the culture media.

Cultural characteristics:

| | | |
|---|---|---|
| Sucrose-nitrate agar | Growth | Grow moderately<br>Light beige (3ec) |
| | Reverse | Pearl pink (3ca) and Light tan (3gc) |
| | Aerial mycelia | Grow moderately<br>White (a) |
| | Soluble pigment | - |
| Glucose-asparagine agar (ISP) | Growth | Grow moderately<br>Pearl pink (3ca) |
| | Reverse | Light ivory (2ca) |
| | Aerial mycelia | Grow moderately<br>Whitish Purple(10ba) |
| | Soluble pigment | - |

| | | |
|---|---|---|
| Glycerol-asparagine agar (ISP) | Growth | Grow moderately with penetration<br>Pearl pink (3ca) |
| | Reverse | Pearl pink (3ca) |
| | Aerial mycelia | Grow moderately<br>White (a) |
| | Soluble pigment | - |
| Starch-inorganic salt agar (ISP) | Growth | Grow well<br>Light ivory (2ca) |
| | Reverse | Light ivory(2ca)and olive gray (lig) |
| | Aerial mycelia | Grow abundantly<br>Outside white (a)<br>Inside silver gray (3fe) |
| | Soluble pigment | - |
| Tyrosine agar (ISP) | Growth | Grow moderately with penetration<br>Pearl pink (3ca) |
| | Reverse | Light beige (3ec) |
| | Aerial mycelia | Grow abundantly<br>Pale blue (13 1/2ca) |
| | Soluble pigment | - |

| | | |
|---|---|---|
| Oatmeal agar (ISP) | Growth | Grow well with penetration<br>Pearl (3ba) |
| | Reverse | Pearl (3ba) |
| | Aerial mycelia | Grow poorly<br>Mustard brown (2pl) |
| | Soluble pigment | - |
| Yeast extract-malt extract agar (ISP) | Growth | Grow well<br>Light wheat (2ea) |
| | Reverse | Bamboo (2gc) |
| | Aerial mycelia | Grow abundantly<br>Silver Gray (3fe) |
| | Soluble pigment | - |
| Nutrient agar | Growth | Grow moderately<br>Light ivory (2ca) |
| | Reverse | Light ivory (2ca) |
| | Aerial mycelia | Grow poorly<br>Pearl pink (3ca) |
| | Soluble pigment | - |
| Peptone yeast-extract agar(ISP) | Growth | Grow moderately<br>Pearl pink (3ca) |
| | Reverse | Colonial yellow (2ga) |
| | Aerial mycelia | Grow poorly<br>Whitish purple (10ba) |
| | Soluble pigment | - |

| | | |
|---|---|---|
| Glucose-nitrate agar | Growth | Grow moderately<br>Light beige (3ec) |
| | Reverse | Pearl pink (3ca) |
| | Aerial mycelia | Grow moderately<br>White (a) |
| | Soluble pigment | - |
| Glycerol-calcium malate agar | Growth | Grow moderately<br>Pale orange (5ca) |
| | Reverse | Pale orange (5ca) |
| | Aerial mycelia | Grow very poorly<br>White (a) |
| | Soluble pigment | - |
| Glucose-peptone agar | Growth | Grow moderately<br>Light brown (4ng) |
| | Reverse | Oak brown (4pi) |
| | Aerial mycelia | Grow poorly<br>White (a) |
| | Soluble pigment | - |

(III) Physiological characteristics:

(1) Melanin formation:

(a) Tyrosine agar negative

(b) Peptone-yeast-iron agar negative

(c) Glucose-peptone-gelatin medium negative (21-23$^{\circ}$C)

(d) Triptone-yeast liquor negative

(2) Tyrosinase reaction negative

(3) Hydrogen sulfide production negative

(4) Reduction of nitrate negative

(5) Liquefaction of gelatin (21-23$^{o}$C) negative
(glucose-peptone-gelatin medium)

(6) Hydrolysis of starch (3 weeks) positive

(7) Coagulation of defatted milk positive

(8) Peptonization of defatted milk negative

(9) Temperature range for growth 13.5-34.2$^{o}$C

(10) Utilization of carbon sources
(Pridham and Gottlieb's agar medium)

Utilized : D-Glucose, L-arabinose, D-xylose, raffinose, melibiose, D-mannitol, D-fructose, i-inositol, sucrose

Not Utilized : L-Rhamnose

(11) Cellulolytic activity negative

(IV) Composition of cell wall:

Diaminopimelic acid in cell wall is LL type.

In summarizing, the mycetological characteristics of the strain are as follows: Diaminopimelic acid in cell wall is of an LL type. The aerial mycelia are spiral in shape and form long spore chains. The spores have smooth surfaces. As cultural characteristics, the vegetative mycelia are beige or pink in color, while the aerial mycelia are white or gray. No soluble pigments are produced.

From the foregoing results, the strain is considered to belong to the genus Streptomyces and to be of the gray

series of the Pridham and Tresner grouping [Bergey's Manual of Determinative Bacteriology 8th Ed., 748-829 (1974)].

The strain was deposited at the Fermentation Research Institute, Agency of Industrial Science and Technology, Ministry of International Trade and Industry, Japan under the name of Streptomyces sp. WK-142 and the accession number of FERM P-8448 on September 13, 1985, and this deposit converted to a deposit under the Budapest Treaty, and the strain has been stored at the FRI under accession number FERM BP-1279. The strain also produces pepstatin A, which is a known acid protease inhibitor, simultaneously.

Mutants of the above-described strain may be used for the process of the present invention as long as they have an ability to produce the compounds WK-142. Other strains belonging to the genus Streptomyces and capable of producing the compounds WK-142 may also be used for the purpose of the present invention.

As the culture medium, there may be used a synthetic or natural medium containing appropriate amounts of carbon sources, nitrogen sources and inorganic substances suitable for culturing normal actionomyces and, if necessary, other nutrients. Any kind of carbon and nitrogen sources may be utilized in the medium as long as they are suited for the strains used. Various carbohydrates such as glucose, glycerol, fructose, maltose, mannitol, xylose, galactose, ribose, starch and hydrolysates thereof, may be used as carbon sources. The carbon sources are preferably used in

an amount of 0.1-5 % based on the culture medium. Organic acids such as gluconic acid, pyruvic acid, lactic acid and acetic acid; amino acids such as glycine, glutamic acid and alanine; alcohols such as methanol and ethanol; non-aromatic hydrocarbons such as normal paraffins; and fatts such as vegetable and animal fats may also be used.

As the nitrogen sources, there may be used ammonium salts of inorganic or organic acids such as ammonia, ammonium chloride, ammonium phosphate, ammonium sulfate and ammonium nitrate; nitrogen-containing organic substances such as urea, peptone, NZ-amine, meat extracts, yeast extracts, dry yeast, corn steep liquor, casein hydrolysates, fish meal or its digests, and defatted soybean or its digests or hydrolysates.

As the inorganic substances, phosphates, magnesium sulfates, salt or trace amounts of heavy metal salts may be used.

When mutants requiring a nutrient are used, it is of course necessary to add a substance capable of satisfying the requirement into the culture medium. In some cases, however, such a nutrient need not be added when the medium used contains a natural product.

The cultivation is generally performed under aerobic conditions utilizing shaking cultivation or aerobic agitation cultivation. Deep aerobic agitation cultivation is preferably adopted in practice. The culture medium has generally a pH of 5.0-8.0, preferably a neutral pH. The

cultivation is performed at a temperature of, for example, 20-40°C, generally 26-32°C, preferably about 27°C, and for a period of generally 1-8 days in the case of liquid state culture. The culture is continued until the amount of the compounds WK-142 accumulated in the culture becomes maximum. The culture conditions such as chemical composition of the medium, pH of the medium, cultivation temperature, agitation temperature and air feed rate are suitabley controlled and selected according to the kind of the strain used and in consideration of ambient conditions. An anti-foaming agent such as silicone oil, vegetable oil or a surfactant may be used, if necessary, in the case of liquid cultivation.

The compounds WK-142 produced and accumulated in the culture are generally present in the filtrate thereof, though about 10 % of the product is sometimes present in the cell pellet.

The compounds WK-142 may be recovered from the filtrate with the use of conventional means customarily utilized for collecting metabolites from micro organism cultures, such as filtration, centrifugation, dialysis, concentration, drying, lyophilization, adsorption, desorption, methods utilizing the difference in solubility (e.g. precipitation, crystallization, recrystallization, extraction and counter current distribution) and chromatography. These techniques are adopted by themselves, in combination in any suitable order, or repeatedly.

Since the compounds WK-142 are produced and accumulated in the filtrate, they are recovered from the culture after separation of the cells therefrom. Chromatography using cation or anion exchangers (e.g. ion exchange resin, Sephadex ion exchanger and ion exchange cellulose), gel filtration materials, activated carbon, high-porous polymers (e.g. Amberlite XAD-2 manufactured by Rohm & Haas Inc), Diaion HP-20 (manufactured by Mitsubishi Chemical Industries Co., Ltd.), alumina, florisil, silicagel, or cellulose, may be advantageously used.

Pharmaceutically acceptable salts of the compounds of the present invention, such as sodium salts, magnesium salts, calcium salts and potassium salts, may be prepared in the conventional manner and the explanation of such a known method is omitted here.

DESCRIPTION OF THE PREFERRED EMBODIMENTS

The present invention is explained below by way of example, but the present invention is not limited thereto.

Example

A liquid medium (pH 7.0) containing 0.1 % glucose, 2.4 % starch, 0.3 % peptone, 0.3 % meat extract, 0.5 % yeast extract, and 0.4 % calcium carbonate was added into each of seven 500 ml Erlenmeyer flasks in an amount of 100 ml and subjected to steam sterilization at 121°C for 15 minutes. In to each liquid medium was then innoculated one platinum loop of stock culture of strain WK-142 which had been cultured at 27°C in a slanting agar medium containing 1.0% glucose, 1.0 % calcium malate, 0.05 % ammonium chloride,

0.05 % dipotassium phosphate, 0.1 % yeast extract and 1.5 % agar. The inoculated medium was incubated on a shaking machine at 27°C for 3 days to obtain a seed culture. A liquid medium (pH 7.0) containing 5.0 % peptone, 0.5 % sodium chloride, 0.001 % ferrous sulfate heptahydrate, 0.001 % manganese chloride tetrahydrate, 0.001 % zinc sulfate heptahydrate, 0.001 % copper sulfate pentahydrate and 0.001 % cobalt chloride was added into a 100 liter jar fermenter in an amount of 70 liters and subjected to steam sterilization at 121°C for 15 minutes. Into the liquid medium thus prepared, the seed culture in the seven flasks obtained above was transferred. The aerobic culture was performed at 27°C for 72 hours with stirring at 200 r.p.m. while feeding air at a rate of 35 liter per minute. The resulting culture liquid was centrifuged at 10000 r.p.m. using a Scharples type centrifuge to obtain a supernatant and cell pellet. The thus obtained supernatant was adjusted to pH 3.0 with hydrochloric acid and extracted with 20 liters of ethyl acetate. The extract was further extracted with 5 liters of 0.1 N $NH_4OH$ to obtain an aqueous layer separated from an organic solvent layer. The aqueous layer was adjusted to pH 2.0 with hydrochloric acid and reextracted with ethyl acetate. The resultant extract was concentrated in vacuo and the concentrate was added into 250 ml of benzene. The resulting precipitates were recovered by filtration. Such concentration and precipitation treatments were repeated three times in total. The precipitates thus

recovered were dried to obtain 896 mg of a crude product. The crude product was dissolved in a small amount of chloroform, applied on a silicagel column and eluted with chloroform: methanol (15:1). The active fractions were combined and evaporated to dryness to obtain 417 mg of an oily meterial. This was dissolved in 30 ml of methanol and mixed with 70 ml of water with stirring. The resulting aqueous solution was divided into 10 equal portions and each portion applied on SEP-PAK (manufactured by Waters Association Inc.) followed by washing with 5 ml of 30 % aqueous methanol and with 5 ml of 50 % aqueous methanol and elution with 80 % aqueous methanol. The eluate was evaporated to dryness. The resultant crude material (29.1 mg) was dissolved in a small amount of acetic acid and divided into 3 portions. Each portion was charged on a preparative reversed phase column for high speed liquid chromatocgraphy (AM324 (ODS) manufactured by Yamamura Chemical Institute, 10x300 mm) and eluted with 42.5 % aqueous acetonitrile containing 0.2 % of acetic acid. The active fractions were combined and evaporated in vacuo to dryness to obtain 3.45 mg of active material A (the compound of the general formula (I) in which X is isobutyl and Y is isobutyl), 0.6 mg of B (the compound of the general formula (I) in which X is benzyl and Y is isobutyl), 1.8 mg of C (the compound of the general formula (I) in which X is isobutyl and Y is n-propyl), and 1.4 mg of D (the compound of the general formula (I) in which X is isobutyl and Y is isopropyl).

0236948

What Is Claimed Is:

(1) Compounds WK-142 of the following general formula:

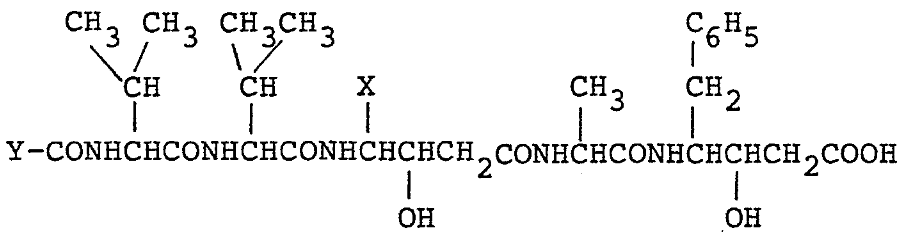

(I)

wherein X stands for a benzyl group or an isobutyl group and Y stands for an alkyl group, or pharmaceutically acceptable salts thereof.

(2) Compounds WK-142 or pharmaceutically acceptable salts thereof according to claim 1, wherein Y is an alkyl group having 1 to 18 carbon atoms.

(3) Compounds WK-142 or pharmaceutically acceptable salts thereof according to claim 2, wherein Y is an alkyl group having 1 to 6 carbon atoms.

(4) Compounds WK-142 or pharmaceutically acceptable salts thereof according to claim 3, wherein an alkyl group as Y is n-propyl, isopropyl or isobutyl.

(5) Compounds WK-142 or pharmaceutically acceptable salts thereof according to claim 1, wherein X is isobutyl and Y is isobutyl.

(6) Compounds WK-142 or pharmaceutically acceptable salts thereof according to claim 1, wherein X is benzyl and Y is isobutyl.

(7) Compounds WK-142 or pharmaceutically acceptable salts thereof according to claim 1, wherein X is isobutyl and Y is isopropyl.

(8) Compounds WK-142 or pharmaceutically acceptable salts thereof according to claim 1, wherein X is isobutyl and Y is n-propyl.

(9) Compounds according to claim 1, wherein pharmacentical acceptable salt is Na, Mg, Ca or K salt.

(10) A process for the preparation of a compound WK-142 of the following general formula:

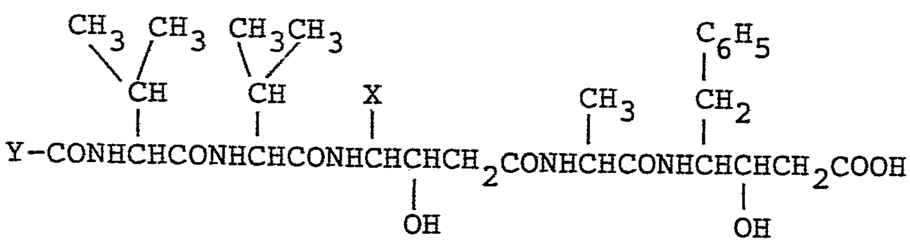

(I)

wherein X stands for a benzyl group or an isobutyl group and Y stands for an alkyl group, said process comprising cultivating in a culture medium a microorganism which belongs to the genus <u>Streptomyces</u> and which is capable of producing the compound WK-142, so that the compound WK-142 is produced and accumulated in the culture, and recovering the compound WK-142 from the culture.

(11) A process according to claim 10, wherein a microorganism is Streptomyces sp. WK-142 (FERM BP-1279).

(12) A process according to claim 10, wherein the cultivation is performed under aerobic conditions.

(13) A process according to claim 12, wherein the cultivation is performed by deep aerobic agitation cultivation.

(14) A process according to claim 10, wherein the culture medium has a pH of 5.0-9.0.

(15) A process according to claim 14, wherein the culture medium has a neutral pH.

(16) A process according to claim 10, wherein the cultivation is performed at 20-40$^{o}$C.

(17) A process according to claim 16, wherein the cultivation is performed at 26-30$^{o}$C.

1/3

0236948

Fig. 1

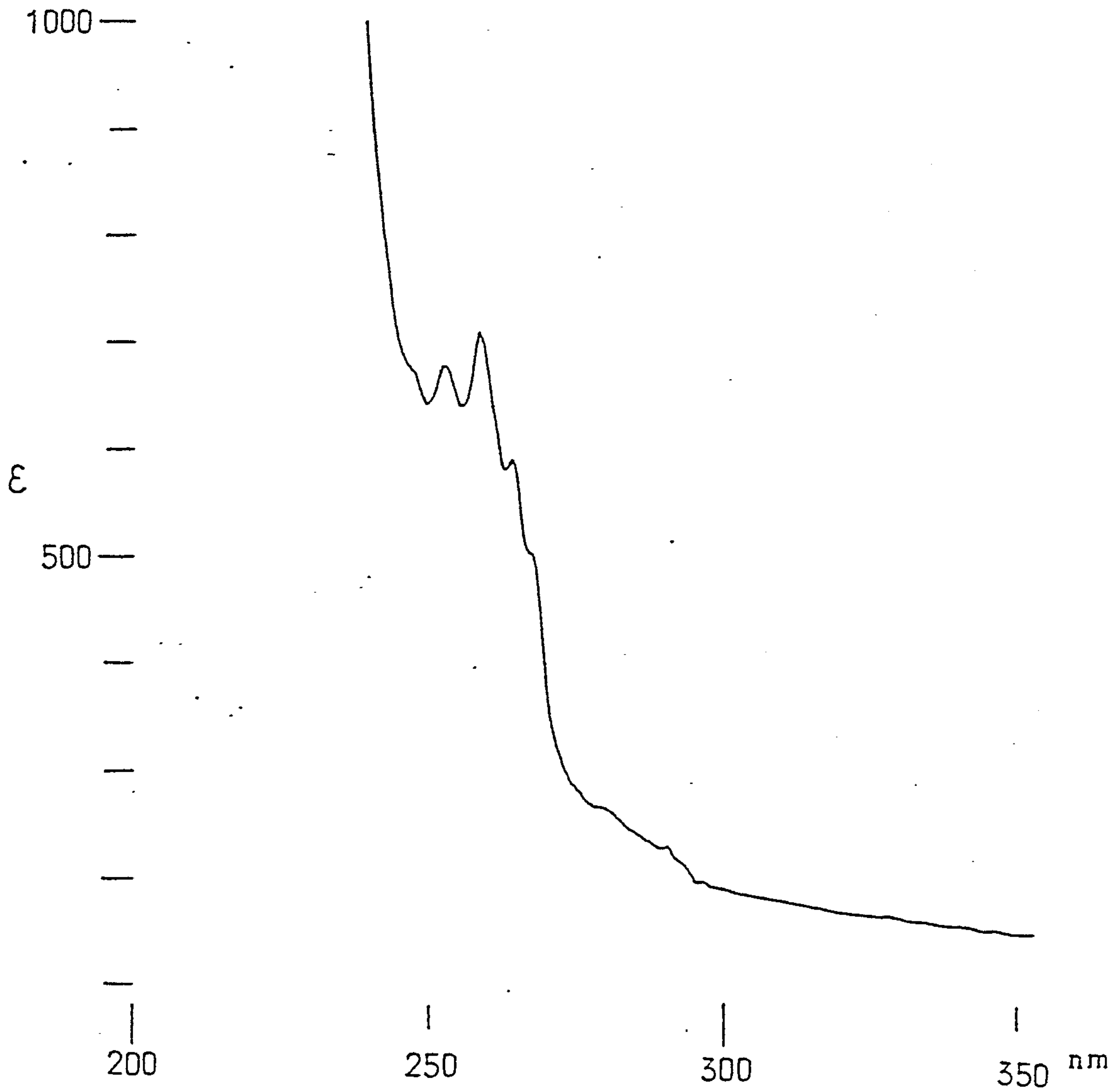

2/3



Fig. 2

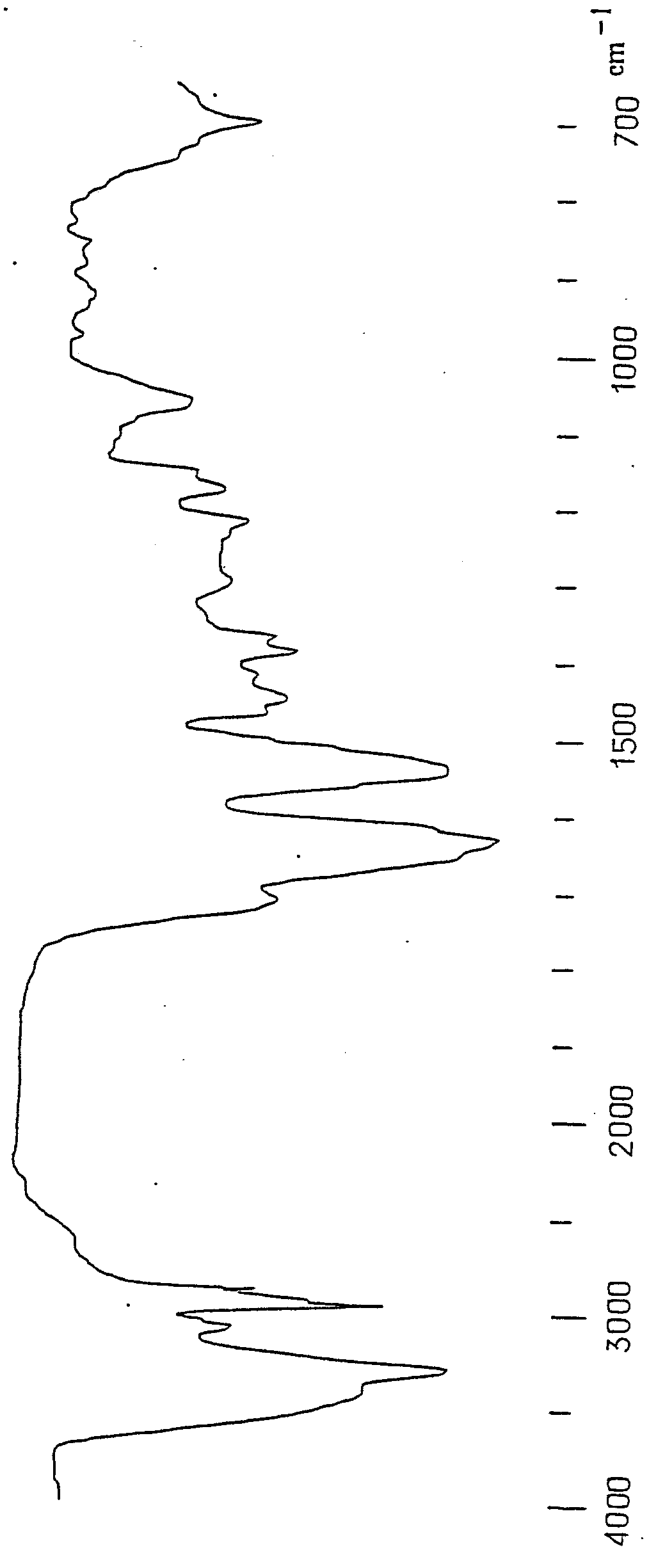

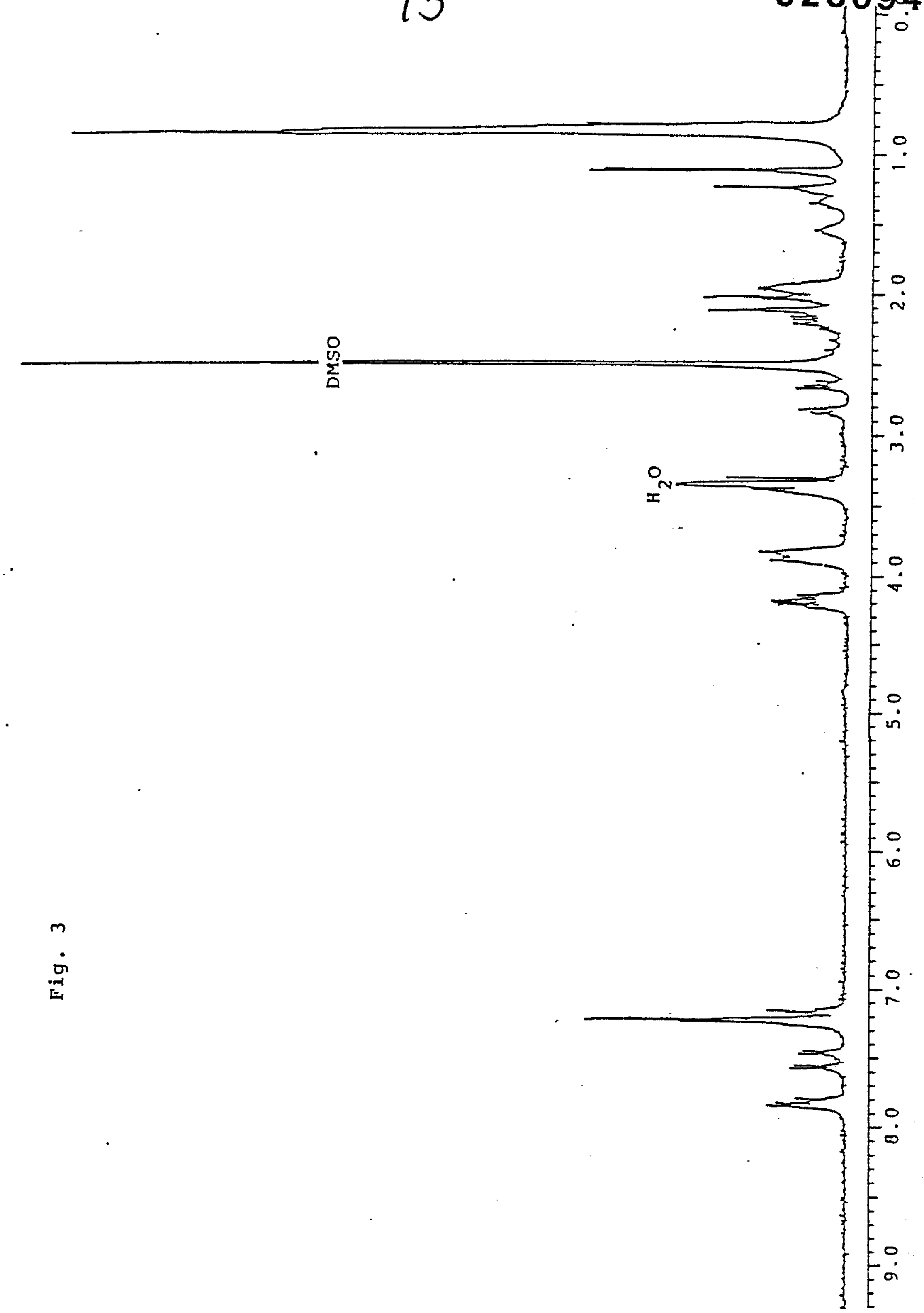


Fig. 3